# EUROPEAN PATENT SPECIFICATION

(11) **EP 0 551 144 B1**
(45) Date of publication and mention of the grant of the patent: **04.06.1997**
(21) Application number: 93102299.0
(22) Date of filing: 18.03.1988
(51) Int. Cl.: C07C 205/12, C07C 201/08

(54) **Process for producing a fluoromononitrobenzene**
Verfahren zur Herstellung von 3-Chlor-4-fluornitrobenzol
Procédé de préparation de 3-Chloro-4-fluoronitrobenzène

(30) Priority: 25.03.1987 JP 68792/87; 14.04.1987 JP 89751/87
(43) Date of publication of application: 14.07.1993
(62) Divisional of application: 88902565.6
(73) Proprietor: NIPPON KAYAKU KABUSHIKI KAISHA, Tokyo 102 (JP)
(72) Inventor: Ishikura, Tsukasa, Ageo-shi, Saitama 362 (JP); Fukushima, Tatuharu, Fukuyama-shi, Hiroshima-ken, 720 (JP)
(74) Representative: Türk, Gille, Hrabal, Leifert

(56) References cited:
- US-A- 2 256 999
- US-A- 4 021 498
- US-A- 4 453 027

## Description

The present invention relates to a process for producing fluoromononitrobenzene. Most specifically, the present invention relates to a process for producing fluoromononitrobenzene having a high purity with a high yield.

Fluoromononitrobenzene is a useful compound as an intermediate for medicine and agricultural chemicals. For example, it can be used for producing 3-chloro-4-fluoronitrobenzene. Fluoronitrobenzene, i.e. 4-fluoronitrobenzene, is a very desirable compound. Therefore, there is a need to improve the process for production of such compound, i.e. to facilitate the procedure and to increase the purity and the yield.

4-Fluoronitrobenzene is used as a starting material in the process of the parent application. 4-Fluoronitrobenzene is produced according to the present invention by directly nitrating fluorobenzene. Nitration of fluorobenzene is more advantageously carried out in sulfuric acid kept in a specific concentration and a temperature in a specific range.

US-A-4,021,498 refers to an adiabatic process for the mononitration of nitratable aromatic hydrocarbons and halogenated aromatic hydrocarbons by batchwise or continuous addition of a mixture of sulfuric acid and nitric acid to the aromatic hydrocarbon at a temperature in the range of 60-70°C or lower.

US-A-2,256,999 describes a method for producing nitrated compounds by reaction of organic compounds with sulfuric acid and nitric acid, wherein the sulfuric acid content of the mixed acid will be between 68 and 76%.

US-A-4,453,027 refers to a process for producing nitrohalobenzenes from halobenzenes by reacting a halobenzene with nitric acid and a second acid selected from sulfuric acid, phosphoric acid or sulfonic acid and mixtures of the above wherein the concentration of sulfuric acid is preferably 77 - 78% by weight.

However, mononitration of fluorobenzene is not disclosed in any of these documents.

Present invention relates to a process for producing a fluoromononitrobenzene, which comprises treating a fluorobenzene with a nitrating agent in sulfuric acid, the concentration (weight bei weight) of said sulfuric acid being kept in a range of 70 to 78% during the reaction at a temperature of 30 to 80°C.

The concentration (weight ratio) of sulfuric acid to be kept during the nitration reaction is selected in the range of 70 to 78%.

The reaction temperature is 30° to 80°C, more preferably 40° to 70°C. As a nitrating agent, nitric acid is preferably used. The amount of nitric acid is preferably 1.0 to 1.1 times of the theoretical amount. As a method of addition of nitric acid, though nitric acid can be added alone, nitric acid is more preferably added in a state of mixed acids composed of nitric acid and sulfuric acid.

The reaction time is usually 0.5 to 5 hours, though it varies depending on the reaction conditions. After completion of the reaction, the sulfuric acid layer is separated from oil layer and used by recycling. The oil layer is washed with warm water, then with an aqueous alkali solution followed by distillation to separate fluoromononitrobenzenes, respectively.

The process for producing 4-fluoronitrobenzene which is a starting material for the preparation of 3-chloro-4-fluoronitrobenzene is illustrated referring to Examples.

### Example

### Process for producing 4-fluoronitrobenzene

### Examples 1 and 2

Three reactors made of SUS were connected in cascade system. Fluorobenzene and a mixed acid (HNO₃-H₂SO₄-H₂O, weight ratios described in the following table) were poured into the first reactor at feeding rates of 94 ml/hr and 210 ml/hr, respectively, by quantitative feeding pumps at 50° to 55°C (retention time: 1.5 hours).

After the reaction, oil layer was separated and washed with warm water, an aqueous alkali solution, then warm water to obtain fluoromononitrobenzenes.

**Table**

| | Composition of mixed acid | | | Conc. of H₂SO₄ during nitration | Yield | Composition | | |
|---|---|---|---|---|---|---|---|---|
| | H₂SO₄ | HNO₃ | H₂O | | | Fluorobenzene | Fluoromononitrobenzenes | Fluorodinitrobenzene |
| Ex.1 | 61.7% | 20.3% | 18.0% | 72.5% | 98% | 0.5 | 99.5 | - |
| " 2 | 63.6 | 20.3 | 16.1 | 75.0 | 98 | 0.4 | 99.6 | - |

The proportions of respective fluoromononitrobenzenes thus obtained in Examples 1 and 2 measured by gas chromatography were shown as follows:

| | p⁻ | o⁻ | m⁻ |
|---|---|---|---|
| Example 1 | 85.0 | 14.7 | 0.3 |
| Example 2 | 85.1 | 14.6 | 0.3 |

Further, 1,000 g of the fluoromononitrobenzenes obtained in Example 1 were subjected to fractionating under reduced pressure to obtain 842 g of p-fluoronitrobenzene (purity: 99.8%) at 86.8° to 87.5°C/19 mm Hg and 137 g of o-fluoronitrobenzene (purity: 99.7%) at 100.7° to 101.2°C/19 mm Hg.

### Example 3

A reactor made of SUS was charged with 48 g of fluorobenzene and 50 g of 72.5% sulfuric acid. Subsequently, a mixed acid (H₂SO₄ : HNO₃ : H₂O = 62.3 : 19.6 : 18.1, weight ratio) was added dropwise at 50° to 55°C, over 40 minutes, then the mixture was stirred for 2 hours. The same succeeding procedures as in Example 1 were repeated to obtain 69.3 g (yield: 98%) of 4-nitromonofluorobenzenes.

Analytical value by gas chromatography:

| | |
|---|---|
| Fluorobenzene | 0.5% |
| Fluoromononitrobenzenes | 99.5% |
| Fluorodinitrobenzene | - |

The proportion of isomers in fluoromononitrobenzenes thus obtained was p- : o- : m- = 85.2 : 14.5 : 0.3.

### Example 4

The same procedures as in Example 3 were repeated, except that 50 g of 75.0% sulfuric acid and 135 g of a mixed acid (H₂SO₄ : HNO₃ : H₂O = 61.0 : 25.4 : 13.6, weight ratio) were used, to obtain 69.1 g (yield: 98%) of fluoromononitrobenzenes.

Analytical value by gas chromatography:

| | |
|---|---|
| Fluorobenzene | 0.3% |
| Fluoromononitrobenzenes | 99.6% |
| Fluorodinitrobenzene | 0.1% |

The proportion of isomers in fluoromononitrobenzenes thus obtained was p- : o- : m- = 85.0 : 14.7 : 0.3.

### Example 5

A reactor made of SUS was charged with 48 g of fluorobenzene and 50 g of 72.5% sulfuric acid. Subsequently, 35.7 g of 96% nitric acid and 139.3 g of 78.3% sulfuric acid were simultaneously added dropwise at 50° to 55°C over 1 hour. The same succeeding procedures as in Example 3 were repeated to obtain 68.8 g (yield: 98%) of fluoromononitrobenzenes.

Analytical value by gas chromatography:

| | |
|---|---|
| Fluorobenzene | 0.4% |
| Fluoromononitrobenzenes | 99.6% |
| Fluorodinitrobenzene | - |

The proportion of isomers in fluoromononitrobenzenes thus obtained was p- : o- : m- = 85.3 : 14.4 : 0.3.

Thus obtained fluoromononitrobenzene may be used for producing 3-chloro-4-fluoronitrobenzene by a process disclosed in the parent application EP 88 902 565.6, for example by the following manner:

### Reference example 1

141 g of 4-fluoronitrobenzene, 3.8 g of ferric chloride and 0.2 g of iodine were charged and then heated. At a temperature of 60° to 70°C, chlorine gas was blown into the liquid until the starting material could not be detected (over 12 hours) to complete the reaction. Subsequently, the reaction mixture was washed three times with 100 g each of warm water followed by liquid separation to obtain 174 g (yield: 99%) of 3-chloro-4-fluoronitrobenzene. Analytical value thereof by gas chromatography was as follows:

| | |
|---|---|
| 4-fluoronitrobenzene | 0.4% |
| 3-chloro-4-fluoronitrobenzene | 98.1% |
| dichloro-4-fluoronitrobenzene | 1.4% |

## Claims

1. The process for producing a fluoromononitrobenzene which comprises treating a fluorobenzene with a nitrating agent in sulfuric acid the concentration (weight by weight) of said sulfuric acid being kept in a range of 70 to 78% during the reaction at a temperature of 30 to 80°C.

2. The process for producing a fluoromononitrobenzene according to Claim 1, wherein said nitrating agent is nitric acid.

## Patentansprüche

1. Verfahren zur Herstellung eines Fluormononitrobenzols, welches Behandlung eines Fluorbenzols mit einem Nitrierungsmittel in Schwefelsäure umfaßt, wobei die Konzentration (Gewicht bezogen auf Gewicht) der Schwefelsäure währen der Reaktion in einem Bereich von 70 - 78 % gehalten wird, bei einer Temperatur von 30 - 80 °C.

2. Verfahren zur Herstellung eines Flourmononitrobenzols entsprechend Anspruch 1, wobei das Nitrierungsmittel Salpetersäure ist.

## Revendications

1. Procédé de préparation d'un fluoromononitrobenzène, selon lequel on traite un fluorobenzène avec un agent de nitration dans de l'acide sulfurique, la concentration (poids/poids) de l'acide sulfurique étant maintenue entre 70 et 78 % au cours de la réaction à une température de 30 à 80 °C.

2. Procédé de préparation d'un fluoromononitrobenzène selon la revendication 1, dans lequel l'agent de nitration est l'acide nitrique.
